# EUROPEAN PATENT SPECIFICATION

(11) **EP 0 914 831 B1**
(45) Date of publication and mention of the grant of the patent: **16.04.2003**
(21) Application number: 98308655.4
(22) Date of filing: 22.10.1998
(51) Int. Cl.: A61K 39/42, C07K 16/02, C07K 16/04, C07K 16/08

(54) **Biological product for preventive or therapeutic oral administration against canine parvovirosis**
Biologisches Produkt zur präventiven oder therapeutischen oralen Verabreichung gegen Parvovirosis im Hund
Produit biologique pour l'administration orale préventive ou thérapeutique contre le parvovirose canin

(30) Priority: 04.11.1997 CZ 348997
(43) Date of publication of application: 12.05.1999
(73) Proprietor: Medipharm CZ, s.r.o., 693 01 Hustopece u Brna (CZ)
(72) Inventor: Mican, Petr, 593 01 Hustopece u Brna (CZ); Stepanek, Jan, 592 56 Zvole nad Perstynem (CZ)
(74) Representative: Lawrence, John Gordon

(56) References cited:
- EP-A- 0 152 270
- EP-A- 0 554 414
- WO-A-94/21284
- WO-A-96/14088
- PATENT ABSTRACTS OF JAPAN vol. 097, no. 002, 28 February 1997 -& JP 08 259462 A (ADTEC KK), 8 October 1996 -& DATABASE WPI Derwent Publications Ltd., London, GB; AN 96-502641 XP002102129 A
- MEUNIER P C ET AL: "Pathogenesis of canine parvovirus enteritis: sequential virus distribution and passive immunization studies." VETERINARY PATHOLOGY, (1985 NOV) 22 (6) 617-24. JOURNAL CODE: XBQ. ISSN: 0300-9858., XP002102128 United States
- Internet page http://www.springboard4health.com/products/colo- strum/colostrum_more.html
- Internet page from American Veterinary Medical Association: www.avma.org/care4pets/canhparv.htm
- Internet page from magazine "A Breed Apart" (ISSN 1084-0621): www.abap.org/parvo.htm

## Description

The invention pertains to a product for the prevention and therapy of canine parvovirosis.

The causative agent of the disease is canine parvovirus type II (CPV-2) known to exist in a single antigenic type that differs principally from the non-pathogenic type I parvovirus (CPV-1). The way infection of dogs by CPV-2 is almost exclusively oral. Owing to the strong affinity of the virus to rapidly propagating somatic cells, the infection concentrates particularly on epithelial cells of the small intestine. Myocardial cells tend to be also attacked by the virus in young pups. The infection induces degeneration and desquamation of enterocytes and exposure of the stroma of intestinal villi resulting in a considerable reduction of the absorption capacity of intestinal mucosa, hyperbiosis, transport if fluids from tissues into the intestinal lumen, dysbalance of intestinal microflora, and, in some cases, bacterial sepsis induced by facultative enteropathogenic germs (Escherichia coli, Klebsiella spp. Pseudomonas spp. and other species and genera). The clinical pattern of canine parvovirosis is characterized by malignant vomiting, severe diarrhoea resulting in dehydration, and sudden death of pups if myocardium is affected.

Apart from oral administration of specific antibodies, no causal therapy of canine parvovirosis is known. Therefore, the treatment of infection dogs concentrates on alleviation of clinical symptoms, support to defence mechanisms of the patient and inhibition of the development of secondary bacterial infections. Specific prophylactics of canine parvovirosis are based on immunization with live or inactivated vaccines.

Treatment of a gastrointestinal disease using antibodies which have been pre-treated with a proteolytic enzyme or which are administered in the presence of a proteolytic enzyme, optionally in conjuntion with one or more probiotic organisms, is disclosed in WO94/21284. JP08259462 discloses a composition containing antibody recovered from egg yolk of a hen immunised with dog parvovirus.

The insufficiency of the current therapeutic and immunoprophylactic methods for canine parvovirosis is generally known. Their cases are summarized below.

Parvovirus is highly resistant to physical factors and chemical agents and survives in the environment in fully active state for prolonged periods.

Infected dogs shedding parvovirus into the environment with faeces are the only source of infection.

Most susceptible to parvovirus infection are pups lacking a fully competent immune system in the early postnatal period. Their resistance to infection depends on antibodies acquired during the embryonic phase (5%) and particularly on colostral antibodies (95%) absorbed during the first three days after birth. In other words, the resistance of pups to parvovirus infection depends on two very variable factors, i.e. the level of maternal immunity (concentration of antibodies to parvovirus in colostrum) and on the amount of colostrum taken during the first post-partum days.

Vaccination of adult dogs does not confer lifelong immunity to parvovirus infection. The immunity reaches its maximum on post-vaccination Days 28 to 35 and decreases thereafter. It is therefore necessary to vaccinate the dogs at least once per year.

The vaccination of pups is complicated by what is called "immune window", i.e. a period of two or four weeks when the level of colostral antibodies decreases below the protective threshold, but is still high enough to neutralize the antigenic effect of the vaccine. During this period, the pups are susceptible to infection but cannot be immunized effectively. The onset of the immune window is individually variable. Generally, it covers the period from 6th to 12th week after birth. Some authors are of the opinion that the period extends up to 16th week. This is apparently the reason why most of the vaccination programmes recommend to start the vaccination at the age of 6 weeks and repeat it at two-week intervals up to the age of 12 to 16 weeks.

The efficacy of specific prophylactics of canine parvovirosis, such as vaccination or oral administration of antibodies, is significantly weaker, because the penetration of circulating antibodies to enterocytes of the small intestine as the primary infection site is rather difficult. The same mechanism limits the efficacy of colostral antibodies absorbed into the circulation.

Owing to its widespread occurrence and dramatic course, canine parvovirosis is regarded as one of the most dangerous canine diseases associated with a high mortality rate, particularly in pups.

The above drawbacks can be removed to a considerable extent by the administration of the oral product for the prevention and therapy of canine parvovirosis as to the invention, containing antibodies to canine parvovirus as the major active component. Further active components include stabilized cultures of lactacidogenic bacteria, such as Enterococcus spp. and/or Lactobacillus spp. with a protective effect, and vitamins with a supportive effect. Homogeneity of the product and standard contents of active components in the administration formulas, such as paste or powder, is ensured by mixing the active components with suitable vehicles. The efficacy of the product as to the invention consists in artificial induction of local immunity of the gastrointestinal mucosa of dogs, particularly pups, to parvovirus infection and in the support of general defence mechanisms of the treated animals. The major active component of the oral product are stabilized specific antibodies to parvovirus that neutralize canine parvovirus in the intestine, inhibit the initial phase of infection consisting in adhesion and penetration of virions into epithelial intestinal cells, and thus inhibit the infection or alleviate its severity.

The antibodies to canine parvovirus are obtained from non-conventional sources including colostrum of cows or eggs of laying hens immunized with the canine parvovirus antigen. The processing of the raw materials, such as bovine colostrum, or, preferably, egg yolks, consists only in careful preservation by spray or fluid drying, or preferably by freeze-drying. The resulting product is a white or yellow powder with a nutritional composition identical with that of feed components commonly used in dog nutrition. The level of antibodies to canine parvovirus in the dried colostrum or egg yolk must reach at least the neutralization titre 16 for 100 TCID₅₀ (50% Tissue Culture Infection Dose) per 0.05 mg dried matter. The biological activity of the antibodies to parvovirus can be enhanced by combining them with a probiotic component ensuring restoration of eubiosis of the intestinal microbial biocenosis impaired by the parvovirus infection. Stabilized live cultures of lactacidogenic genera Enterococcus spp. and/or Lactobacillus spp. can inhibit the propagation of facultatively pathogenic bacteria in the intestine and thus prevent the risk of bacterial sepsis, or reduce it to a minimum. The probiotic component is manufactured by submersive pulsative culture of selected lactacidogenic bacterial strains. After separation from the medium, the cultures are stabilized by freeze-drying. The concentration of live bacteria should reach at least 50 x 10⁹ CFU (Colony Forming Units) per 1 g of the probiotic concentrate. To increase the general resistance to infections, the oral product can be supplemented with a vitamin component containing the vitamins A, C, D, E and group B either in loose form or as an oil suspension. The minimal daily doses for dogs should be 500 I.E. vitamin A, 35 I.E. vitamin D₃, 7 mg vitamin E, 1.2 mg vitamin C, 2 mg niacin, and 0.01 mg biotin. The product is intended largely for oral treatment of pups of various ages that are the highest risk of parvovirus infection. Therefore the following two formulas were chosen. The paste formula, packed in plastic applicators, is suitable for newborn and sucking pups and pups during the milk nutrition period. On the other hand, loose formula (powder or premix) allows easy mixing of the product with any feed type intended for weaned pups and dogs of any age, such as meat homogenates, canned meat, and granulated or extruded feeds. While the paste formula allows safe and accurate oral dosage onto tongue root without any risk of spitting out or unwanted dangerous aspiration, the powder formula is suitable for mixing into feeds for older and/or weaned pups and adult dogs of all age groups. The major components of the vehicle of the paste formula include colloidal silicone-hydrate and distilled monoglycerides that, along with a quality edible oil, form a consistent paste with a molecular mesh ensuring a homogeneous mixing of all active and carrier components. Unwanted microbial contamination of the product must be avoided during all phases of the manufacture of pastes including the filling into the plastic applicators. The final adjustment of the powder formula uses energy-rich carriers, such as glucose, lactose, sorbitol and starch along with dried skim milk or dried whey. The powder formula (pulvis, premix) can be mixed into feeds offered to pups prior to weaning. In order dogs, the product can be added into any feed types such as granulated or extruded feeds and wet meat mixtures.

The major merit of the product as to the invention consists in the possibility to overcome the drawbacks of the currently used preventive and therapeutic methods. Further merits are given below.

The product is manufactured of biological raw materials that are components of conventional dog feeds. It is absolutely non-toxic, hence there is no danger of overdosing even when administered by untrained persons, can be safely administered to pups and adult dogs of any age, can be used both to treat advanced stages ofparvovirus infection and to prevent infection in animals exposed to increased infection pressures, such as those participating in dog shows, competitions, performance tests or housed in dog homes. Its administration can enhance the resistance of pups against parvovirus infection not only during the sucking period and weaning, but also before each transfer into another dog group or before any environmental change. The product as to the invention can also be administered to enhance the resistance of pups during the "immune window" period covering the age of 6 to 12 weeks. The locally administered antibodies do not interfere with the effect of parenteral vaccination and do not block the development of postvaccination immunity to canine parvovirosis. The formulas of the product, such as paste or powder, allow the optimal way of administration to all age categories of dogs eliminating the risk of aspiration in newborn and sucking pups.

### Examples of materialization of the invention

The product for oral prevention and therapy of canine parvovirosis is manufactured by processing the elementary active components, vehicles and vitamin supplements to obtain any of the two basic administration formulas. While the paste is intended for sucking pups during the early postnatal period, the powder formula is intended for mixing into feed for older pups and adult dogs. The elementary active components of the product as to the invention are identical in both formulas.

Canine parvovirus (such as the strain CAPM, V-464, Collection of Animal Pathogenic Microorganisms, Hudcova 70, Brno) propagated in susceptible cell cultures (such as the cell line CRFK, A-72) to reach haemagglutination titre (HT) of 512 to 1024, is used as the immunization antigen for obtaining antibodies to parvovirus. Eventually, the antigen is concentrated ten to twenty times by ultracentrifugation. Pregnant cows or laying hens, treated intramuscularly with 0.2 to 1.0 ml of the virus antigen premixed with an equal volume of an oil adjuvant, are used as antibody donors. The cows are immunized twice before the expected parturition and laying hens are immunized three times at 21-day intervals and subsequently as necessary, usually at three-week or four-week intervals.

The levels of antibodies to parvovirus are checked using haemagglutination inhibition or virus neutralization tests. The antibody titre is expressed in terms of reciprocal values of blood serum, colostrum, or egg suspension dilutions inhibiting 8 agglutination units or neutralizing the infection potential of 100 TCID₅₀ of canine parvovirus.

The concentration of 16/100 TCID₅₀ of antibodies to canine parvovirus per 1 g product is necessary to achieve the expected effect.

Cow's colostrum or egg yolks of the immunized donors must be thoroughly homogenized before further processing. The homogenized colostrum or egg homogenate is eventually preserved by spray or fluid drying, or, preferably, by freeze-drying. The resulting yellowish-white powder is kept in a dry and cold place up to the subsequent processing.

The probiotic concentrate is manufactured by submersion pulsative culture of selected strains of lactacidogenic bacterial species Enterococcus faecium M74 (CCM 6226) or Lactobacillus casei (CCM 3775) to obtain, after the separation of the medium and stabilization of the condensed biomass by freeze-drying, a dry bacterial concentrate containing at least 50 x 10⁹ live cells per 1 g (CFU 50 x 10⁹ per 1 g). The probiotic concentrates are kept in a freezer at -18°C up to further processing.

### Example 1

One of the final administration formulas of the product as to the invention may be a paste prepared by mixing the active components into vehicles ensuring homogeneity and thus the standard content of the former. Individual active components of the product as to the invention, including specific antibodies to canine parvovirus in the form of colostrum or egg yolks collected from immunized donor animals, the probiotic concentrate containing stabilized live lactacidogenic bacteria, and vitamin supplements, are stepwise mixed with the vehicle under vacuum in a special mixer.

**Table 1:**

| Basic composition of the paste formula of the product | |
|---|---|
| **Vehicle Components** | **w/w%** |
| Quality edible oil (such as groundnut oil) | 40 - 50 |
| Colloid Siloid/hydrolysate (CABOSIL) | 4 - 6.5 |
| Distilled monoglycerides | 1 - 3 |

| **Active Components** | |
|---|---|
| Dried colostrum or egg yolks of immunized animals | 18 - 45 |
| Probiotic concentrate CFU 400 x 10⁹ per 1 g (Enterococcus faecium, Lactobacillus casei) | 1 - 2.5 |

| **Vitamin Supplement** | |
|---|---|
| Mixture of vitamin concentrate (vitamins A, D₃, E C, K₃, B₁, B₂, B₆, B₁₂, biotin, niacin) | 4 - 5 |

After thorough homogenization, the product as to the invention is filled into plastic applicators with a volume of 15 to 30 ml. The usual dosage ranges between 0.25 and 2 ml per animal a day.

### Example 2

The final adjustment of the loose-powder (pulvis/premix) formula of the product as to the invention is carried out in standard mixers with horizontally and vertically moving blades to achieve the highest possible homogeneity of the product. To this end, the active components are first thoroughly premixed with a smaller amount of the vehicle and only then the mixer is completed to the full volume.

**Table 2:**

| Basic composition of the power (pulvis/premix) formula of the product | |
|---|---|
| **Vehicle Components** | **w/w%** |
| Dried instant milk or dried whey | 18 - 39 |

| **Active Components** | |
|---|---|
| Glucose, starch, sorbitol | 18 - 39 |
| Dried colostrum or dried egg yolks from immunized donor animals | 20 - 60 |
| Probiotic concentrate (CFU: 400 x 10⁹ per 1 g) (Enterococcus faecium M74 or Lactobacillus casei) | 1 - 2 |

| **Vitamin Supplement** | |
|---|---|
| Vitamin concentrate (A, D₃, E, C, K₃, B₁, B₂, B₆, B₁₂, biotin, cholin) | 1-2 |

After thorough homogenization, the product as to the invention is filled into packages containing 100 to 250 g. The product is added to feeds for pups and dogs; the per day dose is 3 to 20 g, depending on the age, breed and body weight of the animal.

## Claims

1. A product for preventative or therapeutic oral administration against canine parvovirosis **characterised in that** it comprises colostrum of immunised cows and/or egg yolks of immunised laying hens containing antibodies to the canine parvovirus reaching at least a neutralisation titre of 16 for 100 TCID₅₀, and additionally comprising stabilised live cultures of lactacidogenic bacteria.

2. A product according to claim 1, the stabilised live cultures of lactacidogenic bacteria comprising *Enterococcus spp*. and/or *Lactobacillus spp*.

3. A product according to either one of claims 1 or 2, the concentration of stabilised live cultures of lactacidogenic bacteria being in the range of 100 x 10⁶ to 100 x 10⁹ live cells per 1g.

4. A product according to claim 1, additionally comprising vitamins and vehicle substances.

5. A product according to claim 4, the vitamins comprising at least 100 I.E. vitamin A, 10 I.E vitamin D₃, and 2 mg vitamin E.

6. A product according to claim 4, the vehicle components comprising edible oil 45 to 60 w/w%, silicone hydrolysate 4 to 6.5 w/w%, and distilled glyceride 1 to 3 w/w%.

7. A product according to claim 4, the vehicle components comprising glucose and/or saccharose and/or starch 18 to 39 w/w% and dried instant milk and/or dried whey 18 to 39 w/w%.

8. A product according to claim 1, comprising 18 to 60 w/w% of colostrum of immunised cows and/or egg yolks of immunised laying hens.

9. A method for manufacturing a product according to any one of the preceding claims for preventative or therapeutic oral administration against canine parvovirosis comprising the steps of collecting the colostrum of immunised cows and/or egg yolks of immunised laying hens which have been immunised with canine parvovirus antigen, the antibodies to canine parvovirus reaching at least a neutralisation titre of 16 for 100 TCID₅₀, and processing the colostrum and/or egg yolks into a form suitable for administration.

10. A method according to claim 9, wherein the form suitable for administration comprises homogenised colostrum and/or egg yolks containing the antibodies to canine parvovirus.

11. A method according to either one of claims 9 or 10, wherein the processing of the colostrum and/or egg yolks comprises drying.

12. A method according to claim 11, wherein the processing of the colostrum and/or egg yolks comprises freeze-drying.

13. A method according to any one of claims 9-12, wherein canine parvovirus strain CAPM V-464 is used as an antigen for immunisation.

## Patentansprüche

1. Mittel für die präventive oder therapeutische orale Verabreichung gegen das canine Parvovirus, **dadurch gekennzeichnet,**
**dass** es Kolostrum von immunisierten Kühen und/oder Eidotter von immunisierten Legehennen, die Antikörper zum caninen Parvovirus, mit denen mindestens ein Neutralisationstiter von 16 für 100 TDID₅₀ erreicht wird, enthalten, und zusätzlich stabilisierte lebende Kulturen von lactacidogenen Bakterien enthält.

2. Mittel nach Anspruch 1, **dadurch gekennzeichnet, dass** die stabilisierten lebenden Kulturen von lactacidogenen Bakterien Enterococcus spp. und/oder Lactobacillus spp. enthalten.

3. Mittel nach einem der Ansprüche 1 oder 2, **dadurch gekennzeichnet, dass** die Konzentration der stabilisierten lebenden Kulturen von lactacidogenen Bakterien im Bereich von 100 · 10⁶ + 100 · 10⁹ lebenden Zellen pro 1 g liegt.

4. Mittel nach Anspruch 1, **dadurch gekennzeichnet, dass** das Mittel zusätzlich Vitamine und Trägerkomponenten enthält.

5. Mittel nach Anspruch 4, **dadurch gekennzeichnet, dass** die Vitamine zumindest 100 I.E. Vitamin A, 10 I.E. Vitamin D₃ und 2 mg Vitamin E enthalten.

6. Mittel nach Anspruch 4, **dadurch gekennzeichnet, dass** die Trägerkomponenten 45 bis 60 Gew.-% genussfähige Öle, 4 bis 6,5 Gew.-% Siliciumhydrolysat und 1 bis 3 Gew.-% destillierte Glyceride enthalten.

7. Mittel nach Anspruch 4, **dadurch gekennzeichnet, dass** die Trägerkomponenten 18 bis 39 Gew.-% Glukose und/oder Saccharose und/oder Stärke sowie 18 bis 39 Gew.-% getrocknete Instant-Milch und/oder getrocknete Molke enthalten.

8. Mittel nach Anspruch 1, **dadurch gekennzeichnet, dass** 18 bis 60 Gew.-% Kolostrum von immunisierten Kühen und/oder Eidotter von immunisierten Legehennen enthalten ist.

9. Verfahren zur Herstellung eines Mittels nach einem der vorhergehenden Ansprüche zur präventiven oder therapeutischen oralen Verabreichung gegen das canine Parvovirus umfassend die Schritte des Sammeln von Kolostrum von immunisierten Kühen und/oder Eidotter von immunisierten Legehennen mit caninem Parvovirus-Antigen und den Antikörpern zu dem caninen Parvovirus, mit denen mindestens einen Neutralisationstiter von 16 für 100 TCID₅₀ erreicht wird, und Verarbeitung des Kolostrums und/oder Eidotters in eine für die Verabreichung geeignete Form.

10. Verfahren nach Anspruch 9, wobei die für die die Antikörper zu dem caninen Parvovirus enthaltendes Verabreichung geeignete Form homogenisiertes Kolostrum und/oder Eidotter enthält.

11. Verfahren nach einem der Ansprüche 9 oder 10, wobei die Verarbeitung des Kolostrums und/oder Eidotters eine Trocknung umfasst.

12. Verfahren nach Anspruch 11, wobei die Verarbeitung von Kolostrum und/oder Eidotter eine Gefriertrocknung umfasst.

13. Verfahren nach einem der Ansprüche 9 bis 12, wobei der canine Parvovirusstamm CAPM V-464 als Antikörper für die Immunisierung verwendet wird.

## Revendications

1. Produit pour administration orale préventive ou thérapeutique contre la parvovirose canine, **caractérisé en ce qu'**il comprend un colostrum de vache immunisée et/ou des vitellus d'oeufs de poules pondeuses immunisées contenant des anticorps pour le parvovirus canin atteignant au moins un titre de neutralisation de 16 pour 100 TCID₅₀ et additionnellement comprenant des cultures vivantes stabilisées de bactéries lactacidogéniques.

2. Produit selon la revendication 1, les cultures vivantes stabilisées de bactéries lactacidogéniques comprenant *Enterococcus spp.* et/ou *Lactobacillus spp*.

3. Produit selon l'une quelconque des revendications 1 et 2, la concentration en cultures vivantes stabilisées de bactéries lactacidogéniques étant dans le domaine de 100x10⁶ à 100x10⁹ cellules vivantes par gramme.

4. Produit selon la revendication 1, comprenant en outre des vitamines et des véhicules.

5. Produit selon la revendication 4, les vitamines comprenant au moins 100 I.E. de vitamine A, 10 I.E. de vitamine D₃, et 2 mg de vitamine E.

6. Produit selon la revendication 4, les véhicules comprenant 45 à 60% en poids d'une huile comestible, 4 à 6,5% en poids d'hydrolysat de silicone et 1 à 3% en poids de glycéride distillé.

7. Produit selon la revendication 4, les véhicules comprenant 18 à 39% en poids de glucose et/ou de saccharose et/ou d'amidon, et 18 à 39% en poids de lait instantané séché et/ou de lactosérum séché.

8. Produit selon la revendication 1, comprenant 18 à 60% en poids de colostrum de vaches immunisées et/ou de vitellus d'oeufs de poules pondeuses immunisées.

9. Procédé de fabrication d'un produit selon l'une quelconque des revendications précédentes pour administration orale préventive ou thérapeutique contre la parvovirose canine, comprenant les étapes de collecte de colostrum de vaches immunisées et/ou de vitellus d'oeufs de poules pondeuses immunisées, qui ont été immunisées par l'antigène de parvovirus canin, les anticorps pour le parvovirus canin atteignant au moins un titre de neutralisation de 16 pour 100 TCID₅₀, et de traitement du colostrum et/ou des vitellus d'oeufs en une forme appropriée à l'administration.

10. Procédé selon la revendication 9, dans lequel la forme appropriée pour l'administration comprend du colostrum et/ou des vitellus d'oeufs homogénéisés contenant les anticorps pour le parvovirus canin.

11. Procédé selon l'une ou l'autre des revendications 9 ou 10, dans lequel le traitement du colostrum et/ou des vitellus d'oeufs comprend un séchage.

12. Procédé selon la revendication 11, dans lequel le traitement du colostrum et/ou des vitellus d'oeufs comprend un séchage par réfrigération.

13. Procédé selon l'une quelconque des revendications 9-12, dans lequel la souche CAPM V-464 de parvovirus canin est utilisée comme antigène pour l'immunisation.
